# EUROPÄISCHE PATENTANMELDUNG

(11) **EP 0 913 681 A2**
(43) Veröffentlichungstag der Anmeldung: **06.05.1999**
(21) Anmeldenummer: 98120642.8
(22) Anmeldetag: 03.11.1998
(51) Int. Cl.: G01N 21/33, C11D 1/00

(54) **Vorrichtung und Set zum visuellen Erkennen und/oder Darstellen der Verteilung von fluoreszierenden kosmetischen oder dermatologischen Präparaten sowie Zusammensetzung zur topischen Applikation**

(30) Priorität: 03.11.1997 DE 29719497 U; 24.08.1998 DE 19838441
(71) Anmelder: Bartling-Dudziak, Karin, 69469 Weinheim (DE)
(72) Erfinder: Bartling-Dudziak, Karin, 69469 Weinheim (DE)
(74) Vertreter: HOFFMANN - EITLE

(57) **Zusammenfassung**

Die Erfindung betrifft eine Vorrichtung zum visuellen Erkennen und/oder Darstellen der Verteilung von fluoreszierenden kosmetischen oder dermatologischen Präparaten (P), umfassend mindestens ein hohlraumartig ausgestaltetes Gehäuse (2) mit wenigstens einem vor äußeren Lichteinflüssen abschirmbaren Innenraum (4); mindestens eine im Gehäuse (2) vorgesehene Durchgangsöffnung (8, 38), die in den Innenraum (4) mündet; mindestens eine im Innenraum (4) des Gehäuses (2) angeordnete Lichtquelle (10), die ein Licht in einem vorbestimmten Wellenlängenbereich produziert; und mindestens eine im Gehäuse (2) angeordnete Kontrollöffnung (16, 32, 50). Ferner betrifft die Erfindung ein Set zur Kontrolle der Verteilung eines fluoreszierenden kosmetischen oder dermatologischen Präparates. Des weiteren betrifft die Erfindung eine Zusammensetzung zur topischen Applikation umfassend eine Distyrylverbindung und die Verwendung dieser Zusammensetzung zur Applikationskontrolle von Hautkosmetika, dermatologischer Externa und Desinfektionsmitteln

## Beschreibung

Die Erfindung betrifft eine Vorrichtung zum visuellen Erkennen und/oder Darstellen der Verteilung von fluoreszierenden kosmetischen oder dermatologischen Präparaten. Ferner betrifft die Erfindung ein Set zur Kontrolle der Verteilung eines fluoreszierenden kosmetischen oder dermatologischen Präparates. Des weiteren betrifft die Erfindung eine Zusammensetzung zur topischen Applikation umfassend eine Distyrylverbindung und die Verwendung dieser Zusammensetzung zur Applikationskontrolle von Hautkosmetika, dermatologischer Externa und Desinfektionsmitteln.

Fluoreszierende Präparate finden neben dem rein kosmetischen Gebrauch primär in der dermatologischen Diagnostik, Therapiekontrolle und der Prävention von Dermatosen, insbesondere berufsbedingten Dermatosen oder Umweltdermatosen, Anwendung. Die Präparate sind mit einer fluoreszierenden Substanz (in der Regel mit einer Markersubstanz mit sog. sekundärer Fluoreszenz), zum Beispiel wasserlöslichem Porphyrin oder Vitamin-A-Acetat oder dergleichen vermengt. Typische Anwendungsprodukte dieser Art sind zum Beispiel Schutz-, Reinigungs- oder Pflegepräparate, wie zum Beispiel Hautschutzsalben, Sonnencremes, rein medizinische Präparate oder vergleichbare chemische Zusammensetzung. Wird ein solches Präparat mittels einer Lichtquelle, die ein Licht in einem bestimmten Wellenlängenbereich emittiert, angestrahlt, leuchtet die Markersubstanz in einer für sie charakteristischen Eigenfarbe auf. Geeignete Lichtquellen sind beispielsweise UV A-Lichtlampen, unter anderem auch sogenannten Wood-Lampen. Auf die zuvor beschriebene Weise kann die Verteilung eines auf die Gliedmaßen eines Anwenders oder auf ein Trägermaterial aufgetragenen Präparates dargestellt bzw. eine entsprechende Applikationskontrolle vorgenommen werden. Dieses Verfahren ist in der Dermatologie auch als Fluoreszenztechnik bekannt.

In jüngster Zeit gewinnt die Fluoreszenztechnik besonders in der betrieblichen Anwendung als präventive Maßnahme in der Anwendungskontrolle von Hautschutzpräparaten und in der Schulung hautgefährdeter Beschäftigter an Bedeutung. Bislang werden in der Fluoreszenztechnik hauptsächlich Handlampen zum Ausleuchten behandelter Körperteile verwendet, was sich in der Praxis jedoch als unzureichend erweist, da die Bedienung solcher Geräte nicht nur eine zweite Hilfsperson erfordert, sondern aufgrund der manuellen Handhabung auch keine gleichmäßigen und reproduzieren Prüfbedingungen zuläßt, was jedoch besonders zur Kontrolle und Auswertung sowie Dokumentation von Prüfergebnissen wünschenswert wäre.

Das Aufbringen dermatologischer Externa und von Hautkosmetika wie Cremes, Salben, Lotionen, Gelen, Sprays, Ölen, Hydrogelen, Lösungen, Suspensionen oder Pasten auf die Haut dient in der Regel nicht nur kosmetischen Zwecken, sondern auch der Gesundheit beziehungsweise dem Schutz der Haut. So z.B. helfen Feuchtigkeitsemulsionen, die Hornschicht zu durchfeuchten und über einen möglichst langen Zeitraum feucht zu halten; Reinigungsemulsionen, die Haut zu reinigen; Sonnenschutzemulsionen, die Haut vor einem Sonnenbrand zu schützen; und Hautschutzpräparate, die Haut vor schädlichen Einflüssen wie Staub, Schmutz oder organischen Lösungsmitteln zu schützen. Hierbei ist es in der Regel wichtig, die Zusammensetzung korrekt, vollständig und gleichmäßig auf der Haut aufzutragen. So hat sich gezeigt, daß beispielsweise bei der Applikation von Hautschutzsalben gerade dort, wo sich häufig frühe Zeichen eines irritativen Kontaktekzems finden, die Hautschutzsalbe nur unzureichend aufgetragen war.

Auch bei der Verwendung von Desinfektionsmitteln auf der Haut ist eine korrekte Applikation von Bedeutung. Wird ein Desinfektionsmittel nicht korrekt aufgetragen, kann u.U. die gewünschte Schutzwirkung vor Infektionen bzw. der Übertragung von Infektionen nicht erzielt werden.

Es ist daher nach Verfahren gesucht worden, die es ermöglichen, den Anwender solcher Zusammensetzungen wie Hautkosmetika, dermatologischer Externa oder Desinfektionsmitteln zu schulen, die Zusammensetzung gleichförmig und ganzflächig korrekt aufzutragen.

So sind Salben mit einer fluoreszierenden Substanz markiert worden, die anschließend auf beispielsweise die Hände aufgetragen wurden. Die Verteilung dieser Salbe auf der Handoberfläche konnte dann als Fluoreszenz mittels UV-Bestrahlung sichtbar gemacht werden. Als fluoreszierende Substanz zeigte insbesondere Vitamin-A-Acetat vorteilhafte Eigenschaften (Hautarzt 1997, 48: 523-527). Nachteilig bei der Verwendung von Vitamin-A-Acetat ist jedoch insbesondere deren relativ schwache, mit der Haut kontrastarme, gelbliche Fluoreszenz in der Salbe.

Der Erfindung liegt daher gemäß einem ersten Aspekt die Aufgabe zugrunde, eine Vorrichtung zu schaffen, welche die dem Stand der Technik anhaftenden Nachteile möglichst weitgehend vermeidet und die es auf einfache und effektive Art und Weise ermöglicht, die Verteilung eines kosmetischen und dermatologischen Präparates unter möglichst reproduzierbaren Bedingungen zu erkennen und visuell sichtbar zu machen, und zwar insbesondere auf Gliedmaßen eines Anwenders.

Gemäß einem zweiten Aspekt liegt der Erfindung die Aufgabe zugrunde, ein geeignetes Set zur Kontrolle der Verteilung eines fluoreszierenden kosmetischen oder dermatologischen Präparates zu schaffen.

Gemäß einem dritten Aspekt liegt der Erfindung die Aufgabe zugrunde, eine Zusammensetzung zur topischen Applikation mit einem Fluoreszenzmarker bereitzustellen, der eine gegenüber Vitamin-A-Acetat stärkere, insbesondere mit der Haut kontrastreichere Fluoreszenz aufweist.

Die erstgenannte Aufgabe wird gelöst durch eine erfindungsgemäße Vorrichtung mit den Merkmalen des Anspruchs 1.

Diese Vorrichtung zum visuellen Erkennen und/oder Darstellen der Verteilung von fluoreszierenden kosmetischen oder dermatologischen Präparaten, umfaßt mindestens ein hohlraumartig ausgestaltetes Gehäuse mit wenigstens einem vor äußeren Lichteinflüssen abschirmbaren Innenraum, mindestens eine im Gehäuse vorgesehene Durchgangsöffnung, die in den Innenraum mündet und somit einen Zugang zum Gehäuseinneren schafft, mindestens eine im Innenraum des Gehäuses angeordnete Lichtquelle, die ein Licht in einem vorbestimmten Wellenlängenbereich produziert, und mindestens eine im Gehäuse angeordnete Kontrollöffnung.

Obwohl das Gehäuse vorzugsweise kastenartig und im wesentlichen vollkommen geschlossen ausgebildet ist, ist im Sinne der Erfindung unter einen hohlraumartigen Gehäuse auch ein solches Gehäuse zu verstehen, das beispielsweise die Form einer U-förmig ausgestalteten Blende mit zumindest teilweise offenen Seitenflächen besitzt, wobei die Blende einen gegen äußeren Lichteinflüsse im wesentlichen abgeschirmten Innenbereich aufweist. Die Durchgangsöffnung dient dazu, ein mit den fluoreszierenden kosmetischen oder dermatologischen Präparaten behandeltes Objekt oder entsprechend behandelte Gliedmaßen eines Anwenders in vivo in den abgeschirmten Innenraum der Vorrichtung einzuführen und dort mittels der Lichtquelle auszuleuchten. Für das visuelle Erkennen und/oder Darstellen der Verteilung des fluoreszierenden kosmetischen oder dermatologischen Präparates auf den Gliedmaßen eines Anwenders empfiehlt sich besonders eine paarweise Bereitstellung von Durchgangsöffnung. Neben den für ein Testobjekt oder eine Testperson beziehungsweise einen Patienten vorgesehenen Durchgangsöffnungen können bei Bedarf weitere Durchgangsöffnungen beispielsweise für eine Hilfsperson oder für einen behandelnden Arzt sowie für etwaige für eine Behandlung erforderliche Instrumente angebracht sein. Es ist hierbei denkbar, diese zusätzlichen Durchgangsöffnungen in der Art einer aus der chemischen oder medizinischen Praxis bekannten "glove box" mit Arbeitshandschuhen auszukleiden, die gegen den Innenraum hermetisch verschlossen sind. Als Lichtquelle hat sich für ein breites Einsatzspektrum insbesondere eine UV-Lichtquelle bewährt. Die Erfindung ist indes nicht ausschließlich auf diesen Lichtquellentypus beschränkt. Unter bestimmten Voraussetzungen können ebenso andere geeignete Lichtquellen zum Einsatz kommen. Ausreichend ist hierbei, daß abgestimmt auf das jeweils verwendete Präparate der gewünschte Lumineszenzeffekt erzielt wird. Die Lichtquelle kann fest an einer bestimmten Stelle des Kasteninnenraumes installiert oder aber zum Erzielen einer variablen Ausleuchtung beweglich angeordnet und beispielsweise über einen Bewegungsmechanismus motorisch angetrieben sein.

Die Kontrollöffnung dient der Inaugenscheinnahme oder Überwachung des in den Innenraum eingeführten Testobjektes und kann sowohl durch die Testperson selbst und/oder von einem weiteren Beobachter benutzt oder auch separaten Meß- oder Kontrolleinrichtungen zugeordnet werden. Sofern diese Einrichtungen in dem Gehäuse selbst angeordnet sind, kann die Kontrollöffnung gegebenenfalls vollkommen verschlossen oder sogar auf diese verzichtet werden. Ein gleiches gilt, falls die Durchgangsöffnungen selbst derart ausgestaltet sind, daß sie die Funktion der Kontrollöffnung übernehmen können. Die Vorrichtung beziehungsweise deren Teilbereiche ist/sind den jeweiligen technischen, hygienischen, physiologischen und ergonomischen Anforderungen entsprechend aus geeigneten Materialien, z.B. geeigneten licht- und chemikalienbeständigen oder hautverträglichen Werkstoffen, gefertigt und geformt. Die Vorrichtung ist im Hinblick auf die Lichtquelle sowie etwaige andere energieabhängige Zusatzeinrichtungen mit einer internen Energiequelle ausgestattet oder aber über geeignete Adapter mit einer externen Energiequelle verbindbar. Des weiteren verfügt die Vorrichtung zum Zwecke ihrer Aktivierung über eine Schalteinrichtung sowie zur Überwachung ihres Betriebszustandes über geeignete Betriebszustandskontrollinstrumente. Die Schalteinrichtung kann auch berührungslos ausgebildet sein, und hierbei zum Beispiel über einen mit der Schalteinrichtung gekoppelten Annäherungssensor automatisch in Betrieb gesetzt werden.

Die erfindungsgemäße Vorrichtung ermöglicht es auf einfache und effektive Art und Weise unter Ausnutzung der Fluoreszenztechnik das Vorhandensein und die Verteilung eines auf die Oberfläche eines Testobjektes oder die Gliedmaßen eines Anwenders aufgetragenen kosmetischen oder dermatologischen Präparates oder anderer chemischer Substanzen unter reproduzierbaren Bedingungen zu erkennen und visuell sichtbar zu machen, indem das Testobjekt beziehungsweise die Gliedmaßen des Anwenders durch die Durchgangsöffnung oder -öffnungen hindurch in den von äußeren Lichteinwirkungen und damit störenden Einflüssen abgeschirmten Geräteinnenraum eingeführt, dort durch die Lichtquelle gleichmäßig und besonders wirkungsvoll ausgeleuchtet und infolge des durch die Lichteinwirkung ausgelösten Fluoreszenzeffektes sehr gut erkennbar dargestellt wird. Das Vorhandensein und/oder die Verteilung des fluoreszierenden kosmetischen oder dermatologischen Präparaten kann im übrigen auch dann erfolgreich kontrolliert werden, falls sich das aufgetragene Präparat unter einem transparenten Arbeits- oder Schutzhandschuh befindet.

Der Anwender benötigt zur Benutzung der Vorrichtung grundsätzlich keine weitere Hilfsperson. Eine Überprüfung des Vorhandenseins und der Verteilung des verwendeten Präparates kann berührungslos erfolgen, wodurch ein ungewollter Kontakt mit anderen Objekten und ein unbeabsichtigtes Abstreifen des Präparates und damit eine Beeinträchtigung der Wirkung des Präparates wirkungsvoll vermeidbar ist. Über die Kontrollöffnung kann durch den Anwender und/oder bei Bedarf auch durch eine weitere Person oder ein geeignetes Meßsystem sofort die deutlich sichtbare Verteilung des Präparates kontrolliert werden. Die erfindungsgemäße Vorrichtung läßt sich leicht, klein und kompakt ausgestalten und kann sowohl als mobile als auch als stationäre Einheit bereitgestellt werden. Die erfindungsgemäße Vorrichtung ist sowohl in medizinischen als auch industriellen Bereichen vielfältig einsetzbar, so zum Beispiel bei der Anleitung und Kontrolle zur wirksamen oder korrekten Applikation von Hautschutzpräparaten in dermatosegefährdeten Arbeitsbereichen wie etwa in der chemischen Industrie, der Lebensmittelindustrie, in Reinigungsbetrieben, in Friseurbetrieben, in Bäckerbetrieben usw., in der medizinischen und industriellen Ausbildung und Anwendung, wie zum Beispiel im OP-Bereich, in Reinräumen, in der Forschung, bei der medizinischen und kosmetischen Hautpflege, bei der medizinischen Überwachung kurativer dermatologischer Maßnahmen, und letztendlich auch im privaten Anwendungsbereich. Die erfindungsgemäße Vorrichtung gestattet zusätzlich unter Ausnutzung der Fluoreszenzmethode die Anwendung und praktische Umsetzung eines sehr anschaulichen und didaktisch besonders geeigneten Verfahrens, um hautgefährdete Beschäftigte zum korrekten Hautschutz zu motivieren.

Die der Erfindung zugrundeliegende erstgenannte Aufgabe wird des weiteren gelöst durch eine erfindungsgemäße Vorrichtung mit den Merkmalen des Anspruchs 2. Diese Vorrichtung besitzt ebenfalls die bereits im Zusammenhang mit der Vorrichtung gemäß Anspruch 1 erläuterten Vorteile. Aufgrund der Phosphoreszenz des Präparates ist der für den Anwender sichtbare Licht- und Erkennungseffekt zwar etwas geringer als bei einem fluoreszierenden Präparat, für viele Anwendungen ist dies jedoch noch völlig ausreichend.

Ferner wird die der Erfindung zugrundeliegende erstgenannte Aufgabe gelöst durch eine erfindungsgemäße Vorrichtung mit den Merkmalen des Anspruchs 3. Diese Vorrichtung besitzt ebenfalls die bereits im Zusammenhang mit der Vorrichtung gemäß Anspruch 1 erläuterten Vorteile. Aufgrund der strahlungsabsorbierenden, nicht-lumineszierenden Eigenschaften des verwendeten Präparates ist der für den Anwender sichtbare Effekt lediglich genau umgekehrt wie bei den zuvor genannten lumineszierenden Präparaten. Das heißt, Stellen, die mit dem kosmetischen oder dermatologischen Präparat bedeckt sind, treten bei Betrachtung dunkler hervor als stellen, die nicht mit dem Präparat bedeckt sind.

Bevorzugte und vorteilhafte Ausgestaltungsmerkmale der erfindungsgemäßen Vorrichtung gemäß den Ansprüchen 1, 2 und 3 sind Gegenstand der Unteransprüche 4 bis 28.

Die der Erfindung gemäß dem o.g. zweiten Aspekt zugrundeliegende Aufgabe wird gelöst durch ein Set zur Kontrolle der Verteilung eines fluoreszierenden kosmetischen oder dermatologischen Präparates, das die Merkmale des Anspruchs 29 aufweist. Ein solches Set bietet ebenfalls die bereits zuvor eingehend erläuterten Vorteile. Anstelle des fluoreszierenden Präparates kann natürlich grundsätzlich auch ein phosphoreszierendes oder ein strahlungsabsorbierendes, nicht-lumineszierendes Präparat Bestandteil des Sets sein. Des weiteren könnte das Set im Sinne der Erfindung zwei oder mehrere dieser Präparate enthalten, um beispielsweise für jeden spezifischen Anwendungsfall ausgerüstet zu sein.

Die der Erfindung gemäß dem o.g. dritten Aspekt zugrundeliegende Aufgabe wird gelöst durch eine Zusammensetzung zur topischen Applikation umfassend eine Distyrylverbindung der folgenden Formel (I): 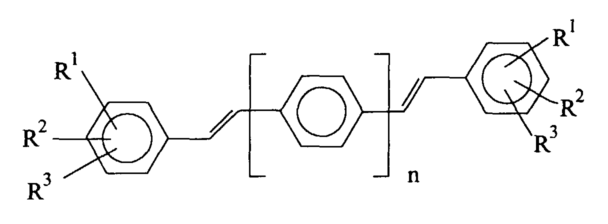 wobei R¹ und R² jeweils unabhängig voneinander ein Wasserstoffatom, ein Halogenatom oder eine C₁-C₄-Alkylgruppe darstellen; R³ ein Wasserstoffatom, ein Halogenatom, eine C₁-C₄-Alkylgruppe, eine Cyanogruppe, SO³⁻M^{+,} wobei M⁺ ein Alkalimetallion darstellt, oder -X-Y-Z darstellt, wobei X eine Einfachbindung, ein Sauerstoffatom, ein Schwefelatom, -CONR₆ oder -COO- darstellt, wobei R₆ ein Wasserstoffatom, eine C₁-C₄-Alkylgruppe, oder Cyanoethyl darstellt, Y C₁-C₄-Alkylen oder Hydroxypropylen darstellt und Z eine quartäre Ammonium-Gruppe darstellt; und n 1 oder 2, vorzugsweise 2 ist. Diese Zusammensetzung hat sich als besonders geeignet für ein fluoreszierendes kosmetisches oder dermatologisches Präparat im Sinne der Erfindung herausgestellt.

Bevorzugte Ausführungsbeispiele der Erfindung mit zusätzlichen Ausgestaltungsdetails und weiteren Vorteilen sind nachfolgend unter Bezugnahme auf die beigefügten Zeichnungen näher beschrieben und erläutert. Es zeigt:
- Fig. 1: eine schematische, teilweise geschnittene Perspektivansicht einer ersten Ausführungsform der erfindungsgemäßen Vorrichtung,
- Fig. 2: eine schematische seitliche Schnittansicht der Vorrichtung von Fig. 1,
- Fig. 3: eine schematische seitliche Schnittansicht einer zweiten Ausführungsform der erfindungsgemäßen Vorrichtung,
- Fig. 4: eine Prinzipskizze einer Schließ- und Öffnungseinrichtung der Vorrichtung von Fig. 3, und
- Fig. 5: eine schematische Frontalansicht einer dritten Ausführungsform der erfindungsgemäßen Vorrichtung.

In der nachfolgenden Beschreibung und in den Figuren werden zur Vermeidung von Wiederholungen gleiche Bauteile und Komponenten auch mit gleichen Bezugszeichen gekennzeichnet, sofern keine weitere Differenzierung erforderlich ist.

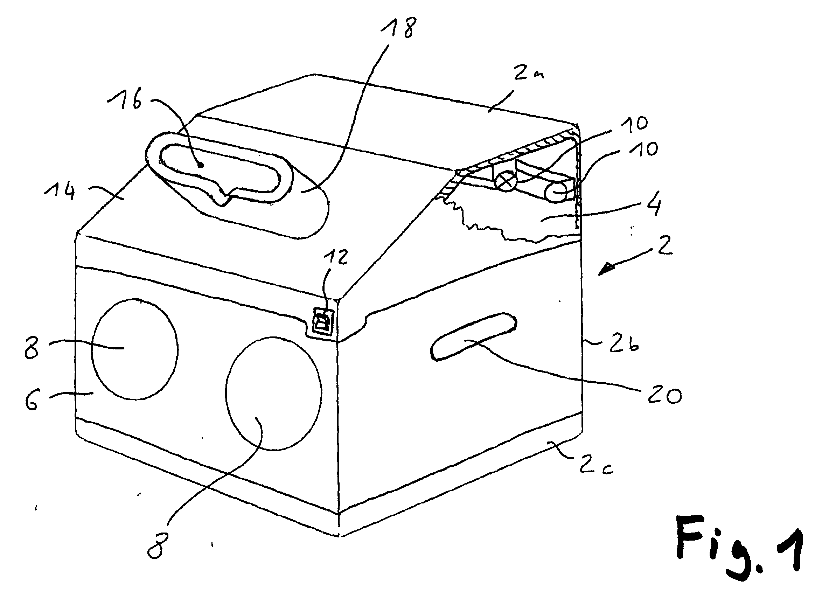

In der Fig. 1 ist in einer schematischen, teilweise geschnittenen Perspektivansicht eine erste Ausführungsform einer erfindungsgemäßen Vorrichtung zum visuellen Erkennen und/oder Darstellen der Verteilung von fluoreszierenden kosmetischen oder dermatologischen Präparaten P gezeigt. Die Vorrichtung besitzt ein hohlraumartig ausgestaltetes, kastenförmiges und aus licht- und chemikalienbeständigen und leicht zu reinigenden Werkstoffen hergestelltes Gehäuse 2 mit einem vor äußerem Lichteinflüssen abschirmbaren Innenraum 4. An einem unteren vertikalen Frontbereich 6 des Gehäuses 2 sind zwei voneinander beabstandete runde Durchgangsöffnungen 8 vorgesehen, die in den abgeschirmten Innenraum 4 münden und somit von außen einen Zugang zum Gehäuseinneren schaffen. Diese Durchgangsöffnungen 8 sind im vorliegenden Fall als Einführungsöffnungen 8 für menschliche Gliedmaßen, nämlich Hände H, ausgebildet. Die Größe der Einführungsöffnungen 8 ist der Größe der zu betrachtenden menschlichen Gliedmaßen H jeweils entsprechend angepaßt, so daß beispielsweise beim Einführen einer Hand H in den Gehäuseinnenraum 4 die Durchgangsöffnung 8 durch die sich an die Hand H anschließende Extremität selbst im wesentlichen lichtdicht verschlossen wird und äußere Lichteinflüsse von dem Innenraum 4 im nahezu vollständig abgeschirmt sind. Im Innenraum 4 des Gehäuses 2, d.h. genauer in einem oberen und hinteren Gehäusebereich, sind zwei Lichtquellen 10 angeordnet, die ein langwelliges UV A-Licht in einem Wellenlängenbereich von ca. 340 bis 459 nm produzieren. Die UV-Lichtquellen 10 sind über nicht dargestellte Leitungen mit einer externen Energiequelle verbunden und können über eine am Gehäuses angeordnete manuelle und/oder automatische Schalteinrichtung 12 an- und ausgeschaltet werden. Wie in der Fig. 1 des weiteren zu erkennen, ist in einem sich an den unteren Frontbereich 6 anschließenden oberen abgeschrägten Frontbereich 14 des Gehäuses 2 eine als Sichtöffnung ausgestaltete Kontrollöffnung 16 vorgesehen, durch die ein Anwender in das ausgeleuchtete Gehäuseinnere 4 blicken kann. Die Sichtöffnung 16 ist sowohl von den beiden Einführungsöffnungen 8 als auch den Lichtquellen 10 beabstandet, so daß der Kasteninnenraum 4 und die durch die Einführungsöffnungen 8 in den Innenraum 4 eingeführten Gliedmaßen H gut von dem Beobachter einsehbar sind. Die Sichtöffnung 16 ist im vorliegenden Fall mit einer tubusartigen Verkleidung 18 ausgestattet und besitzt eine an die Gestalt eines menschlichen Stirn- , Augen- und Nasenbereiches angepaßte äußere Randform. Blickt ein Betrachter durch die Sichtöffnung 16 in den Geräteinnenraum 4, liegt dabei der Rand der Sichtöffnungsverkleidung 18 an den die Augen eines Betrachters umgebenden Kopfbereichen eng an, so daß der Innenraum 4 hermetisch gegen äußere Lichteinflüsse abgeschirmt wird.

Das Gehäuse 2 der Vorrichtung nach Fig. 1 ist modulartig aus drei Teilen bzw. Gehäuseabschnitten 2a - 2b aufgebaut, die lösbar miteinander verbunden sind. Hierbei sind die Lichtquellen 10, die Sichtöffnung 16 und die Schalteinrichtung 12 in einem oberen Gehäuseteil 2a und die Einführungsöffnungen 8 in einem mittleren Gehäuseteil 2b angeordnet, an das sich ein als Bodenabschluß ausgestaltetes drittes, unteres Gehäuseteil 2c anschließt. Das untere Gehäuseteil 2c kann gegebenenfalls weggelassen werden, das heißt das Gehäuse 2 kann nach unten offen ausgestaltet sein, so daß es leicht über einen Gegenstand stülpbar ist. Hierbei ist jedoch zu berücksichtigen, daß auch an dem unten offenen Rand des dritten Gehäuseteiles 2c ein lichtdichter Abschluß gegeben sein sollte. Diese Ausführungsform kann für bestimmte Anwendungsfälle vom Vorteil sein. Des weiteren ist die Vorrichtung mit jeweils an den Seitenflächen des Gehäuses 2 angebrachten Griffen 20 ausgestattet, so daß die Vorrichtung leicht transportierbar ist. Obwohl die Vorrichtung gemäß dem vorliegenden Ausführungsbeispiel als mobile Einheit ausgebildet ist, ist es natürlich ebenso möglich, die Vorrichtung als stationäres Gerät auszugestalten und/oder beispielsweise auf einem Tisch, an einem höhenverstellbaren Ständer oder an einer entsprechenden Halterung oder dergleichen zu fixieren.

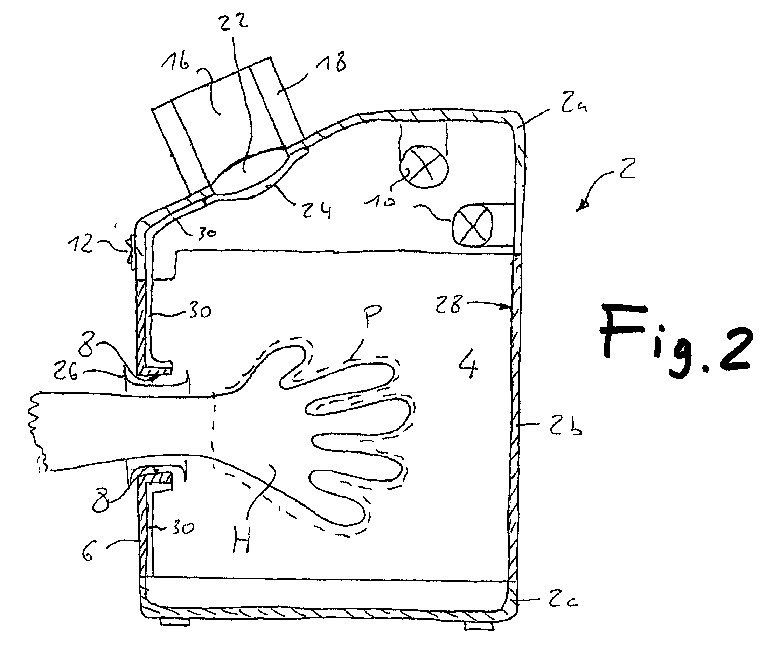

Weitere Details der erfindungsgemäßen Vorrichtung sind in der Fig. 2 dargestellt, die eine seitliche Schnittansicht des in Fig. 1 illustrierten Gerätes zeigt. Wie in der Fig. 2 zu erkennen, ist die Sichtöffnung 16 durch ein Schauglas 22 verschlossen, das im vorliegenden Fall als optisches Instrument in der Form einer leicht vergrößernden Lupe 22 ausgelegt ist. Anstelle einer Lupe 22 sind selbstverständlich auch andere Ausführungsformen möglich. So ist es etwa denkbar die Sichtöffnung 16 durch ein einfaches planes Schau-/Schutzglas abzudecken, oder aber andere optische Instrumente, wie z.B. bestimmte Okulare, Stereoskope oder sogar Fernbeobachtungsvorrichtungen, in die Sichtöffnung 16 zu integrieren. Zur Vermeidung von Augenschäden durch das im Gehäuseinneren 4 ausgestrahlte UV-Licht ist dem Schauglas 22 eine UV-Lichtschutzfiltereinrichtung 24 vorgeschaltet. Der Einsatz von weiteren Lichtfiltereinrichtungen, beispielsweise zum Erzielen von bestimmten optischen Effekten, ist ebenfalls vorgesehen. Diese Filterarten sind in der Zeichnung der Übersichtlichkeit halber jedoch nicht dargestellt.

Die tubusartigen Einführungsöffnungen 8 der Vorrichtung sind mit auswechselbaren hygienischen Einwegmanschetten 26 ausgekleidet, die ein leichtes Austauschen und somit eine hygienische Benutzung der Vorrichtung durch eine Vielzahl von Anwendern gestatten. Anstelle eines Einwegproduktes kann natürlich auch ein zu reinigendes Mehrwegprodukt eingesetzt werden. Grundsätzlich ist es natürlich auch möglich, die tubusartige Verkleidung 18 der Sichtöffnung 16 als auswechselbares hygienisches Ein- oder Mehrwegprodukt auszugestalten.

Gemäß dem vorliegenden Ausführungsbeispiel ist der Innenraum 4 der Vorrichtung im wesentlichen vollständig mit einer schwarzen matten Oberfläche 28 versehen, was unter Berücksichtigung der verwendeten UV-Lichtquellen 10 in Verbindung mit den durch diesen zu erzielenden Fluoreszenz-Effekten gute Sichtverhältnisse gestattet. An den beiden Lichtquellen 10 abgewandten Bereichen des Gehäuseinnenraumes 4 sowie an durch die Sichtöffnung 16 nicht direkt einsehbaren Stellen ist der Geräteinnenraum 4 mit reflektierenden Oberflächen 30 oder speziellen Reflektoren versehen, so daß eine möglichst optimale Ausleuchtung der in den Gehäuseinnenraum 4 gehaltenen Gliedmaßen H realisierbar ist.

Nachfolgend wird nun der Gebrauch bzw. die Verwendung der erfindungsgemäßen Vorrichtung zur Kontrolle der Verteilung eines fluoreszierenden kosmetischen oder dermatologischen Präparates an mit diesem Präparat P eingecremten Händen H beschrieben werden. Ein solcher Gebrauch bzw. eine solche Verwendung erfolgt im Sinne der Erfindung beispielsweise im Rahmen einer Applikationskontrolle dermatologischer Externa, einer Applikationskontrolle von Hautkosmetika, zur Schulung des korrekten Waschens der Haut oder zur Schulung des korrekten Desinfizierens der Haut.

Im vorliegenden Fall wird als fluoreszierendes kosmetisches oder dermatologisches Präparates P eine Zusammensetzung zur topischen Applikation verwendet, umfassend eine Distyrylverbindung der Formel 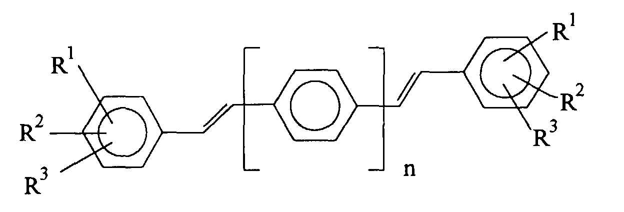 wobei R¹ und R² jeweils unabhängig voneinander ein Wasserstoffatom, ein Halogenatom oder eine C₁-C₄-Alkylgruppe darstellen; R³ ein Wasserstoffatom, ein Halogenatom, eine C₁-C₄-Alkylgruppe, eine Cyanogruppe, SO₃⁻M^{+,} wobei M⁺ ein Alkalimetallion darstellt, oder -X-Y-Z darstellt, wobei X eine Einfachbindung, ein Sauerstoffatom, ein Schwefelatom, -CONR₆ oder -COO- darstellt, wobei R₆ ein Wasserstoffatom, eine C₁-C₄-Alkylgruppe, oder Cyanoethyl darstellt, Y C₁-C₄-Alkylen oder Hydroxypropylen darstellt und Z eine quartäre Ammonium-Gruppe darstellt; und n 1 oder 2, vorzugsweise 2 ist. Diese Zusammensetzung ist in Verbindung mit der erfindungsgemäßen Vorrichtung und den damit zu erzielenden Effekten und Anwendungen besonders geeignet.

Zur Kontrolle der Verteilung des Präparates P an den mit diesem Präparat P eingecremten Händen H, steckt der Anwender seine eingecremten Hände H durch die Einführungsöffnungen 8 hindurch in den Gehäuseinnenraum 4, wo die Hände H von den UV-Lichtquellen 10 direkt und/oder indirekt angestrahlt werden. Infolge des UV-Lichtes leuchten die fluoreszierenden Bestandteile des Präparates P in einer für die charakteristischen Eigenfarbe auf und machen die Verteilung des Präparates P auf der Haut der Hand H sichtbar. Der Anwender selbst kann die Applikation des Präparates P direkt durch die Sichtöffnung 16 überprüfen, wobei die Beobachtungsverhältnisse infolge des im wesentlichen vollständig abgeschirmten Gehäuseinnenraumes 4 hervorragend sind. Nicht oder unzureichend bedeckte Hautbereiche, die bei Händen H erfahrungsgemäß insbesondere an den Fingerendgliedern und in den Fingerzwischenräumen liegen, sind sofort an der fehlenden Fluoreszenz erkennbar und können von dem Anwender nachbehandelt und auf die zuvor beschriebene Weise erneut kontrolliert werden. Der Anwender kann die erfindungsgemäße Vorrichtung, nachdem sie einmal eingeschaltet ist, ohne die Hilfe einer zweiten Person bedienen, und zwar auch dann, wenn er seine zu untersuchenden Gliedmaßen H bereits mit dem jeweiligen kosmetischen oder dermatologischen Präparat P behandelt hat und dementsprechend eine Berührung mit weiteren Gegenständen, insbesondere der Vorrichtung selbst, vermeiden will beziehungsweise muß.

Etwaige von den Händen H herabtropfende Reste des Präparates P oder anderer Substanzen oder dergleichen werden in dem unteren Gehäuseteil 2c aufgefangen, das gleichzeitig als wannenartiger Sammel- oder Auffangbehälter ausgebildet ist.

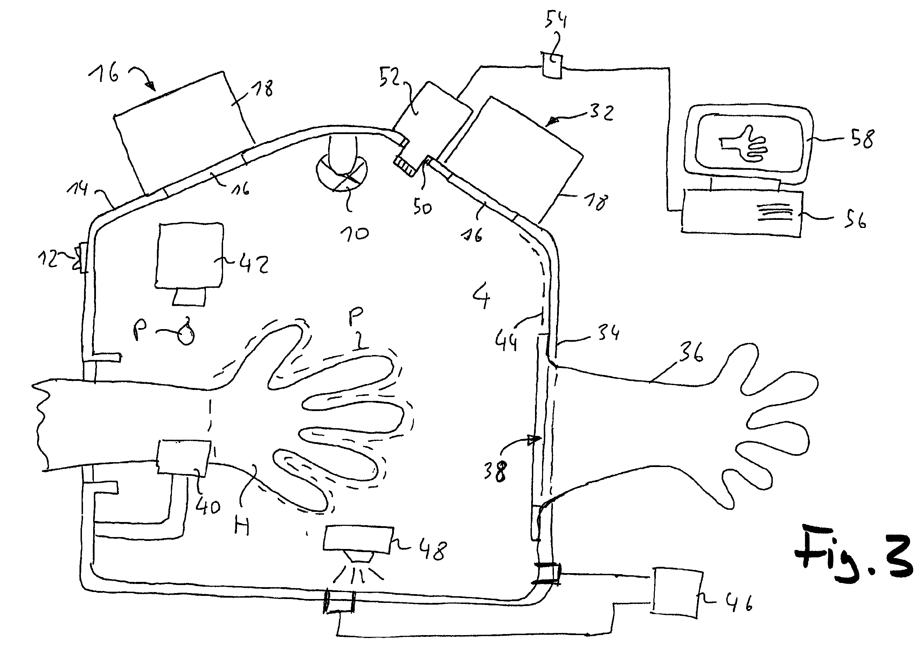

In Fig. 3 ist in einer schematischen Schnittansicht eine zweite Ausführungsform der erfindungsgemäßen Vorrichtung dargestellt. Diese Variante gleicht im wesentlichen der nach den Fig. 1 und 2, ist im Gegensatz dazu jedoch für die Benutzung durch zwei Anwendern ausgelegt, und zwar im vorliegenden Fall für einen Patienten und einen behandelnden Arzt. Gemäß der Darstellung in Fig. 3 ist hierbei die linke Gehäusehälfte dem Patienten und die rechte Gehäusehälfte dem Arzt zugeordnet. Die linke Gehäusehälfte gleicht dementsprechend im wesentlichen der Ausführungsform gemäß Fig. 1 und 2. An der rechten Gehäusehälfte hingegen ist im vorliegenden Beispiel in einem rechten oberen Gehäusebereich eine zweite Sichtöffnung 32 für den Arzt vorgesehen. Des weiteren sind an einem rechten unteren Rückseitenbereich 34 des Gehäuses 2 zwei mit hermetisch gegen den Gehäuseinnenraum 4 abgeschlossenen Handschuhen 36 ausgestattete Durchgangsöffnungen 38 vorgesehen, durch die die behandelnde Person hindurchgreifen und im Gehäuseinnenraum 4 erforderliche Tätigkeiten durchführen kann. In der Fig. 3 sind die Handschuhe 36 der Übersichtlichkeit halber nach außen gestülpt dargestellt. An der Patientenseite des Gehäuseinnenraums 4 ist ferner eine Ablage- und/oder Stütz- und/oder Fixierungs- und/oder Halterungseinrichtung 40 vorgesehen und im wesentlichen in einem von den Lichtquellen 10 erfaßten Strahlungsbereich positioniert, so daß dieser Bereich durch beide Sichtöffnungen 16, 32 hindurch, d.h. sowohl von dem Patienten als auch dem Arzt, gut einsehbar ist. Der Patient kann bei einer längeren Behandlung die zu behandelnden Gliedmaßen an der Einrichtung 40 ermüdungsfrei abstützen oder fixieren. Der Ablage-, Stütz- , Fixierungs- oder Halterungsbereich dieser Einrichtung 40 sollte zweckmäßigerweise so gewählt sein, daß die Applikation des verwendeten Präparates P auf den behandelnden Gliedmaßen H nicht beeinträchtigt wird.

In einem seitlichen Bereich des Gehäuseinnenraumes 4, so daß die Ausleuchtung des Innenraumes 4 sowie die Einsehbarkeit des den zu behandelnden Gliedmaßen H zugeordneten Bereiches des Innenraumes nicht behindert wird, ist eine dosierfähige Spendereinrichtung 42 für das kosmetische oder dermatologische Präparat P oder andere Substanzen vorgesehen, so daß der Anwender selbst oder aber der behandelnde Arzt z.B. nach dem Erkennen einer unzureichenden Verteilung des ursprünglich auf die Gliedmaßen H aufgetragenen fluoreszierenden oder dermatologischen Präparates P eine entsprechende Nachbehandlung bzw. Applikationsänderung vornehmen kann. Der Innenraum 4 der Vorrichtung nach Fig. 3 ist teilweise oder vollständig mit einer auswechselbaren hygienischen Innenauskleidung 44 versehen. Hierbei kann es sich wiederum um ein Einwegprodukt oder aber um ein Mehrwegprodukt handeln. Denkbar sind z.B. saugfähige, waschbare und/oder desinfizierbare und/oder auskochbare und/oder sterilisierbare Textilien oder Einwegtücher oder dergleichen. Der Übersichtlichkeit halber ist die Innenauskleidung 44 in der Fig. 3 nur an einem Teilbereich des Gehäuseinnenraumes 4 mittels einer gestrichelten Linie angedeutet. An den Innenraum 4 ist ferner eine Absaugeinrichtung 46 für flüssige und gasförmige Substanzen angeschlossen. Auf diese Weise können wiederum Reste des kosmetischen oder dermatologischen Präparates P aus dem Innenraum 4 entfernt oder beispielsweise durch Anlegen eines gewissen Unterdruckes ein Austreten von flüssigen und/oder gasförmigen Substanzen aus dem Innenraum 4 effektiv verhindert werden. In der vorliegenden Variante ist die Vorrichtung überdies mit einer Reinigungs- und/oder Desinfektionseinrichtung 48 zum Reinigen oder Desinfizieren des Innenraumes 4 ausgestattet.

Als besonderes Ausgestaltungsmerkmal weist die Vorrichtung nach Fig. 3 zusätzlich eine dem Innenraum 4 zugeordnete und in diesen durch eine weitere Kontrollöffnung 50 mündende Bilderfassungseinrichtung 52 auf, mit der beispielsweise die Applikationskontrolle bzw. Behandlung in dem Gehäuseinnenraum 4 bildlich erfaßt und zu Schulungs- oder Dokumentationszwecken und dergleichen aufgezeichnet werden kann. Die Bilderfassungseinrichtung 52, bei der es sich im vorliegenden Fall um eine Videokamera 52 oder dergleichen handelt, ist zu diesem Zweck über eine geeignete Schnittstelle 54 mit einer externen Datenverarbeitungseinrichtung 56 verbunden, die wiederum mit einer visuellen Darstellungsvorrichtung 58, z.B. einem oder mehreren Monitoren 58 oder Projektoren gekoppelt ist. Die Bilddaten der Bilderfassungseinrichtung 52 können in der Datenverarbeitungseinrichtung 46 gespeichert sowie bei Bedarf weiterbearbeitet werden. Die Bilderfassungseinrichtung 52 dient bei dem Ausführungsbeispiel gleichzeitig als Meßeinrichtung zum automatisierten Messen der Verteilung des kosmetischen und dermatologischen Präparates P, wobei die Meßergebnisse mittels eines geeigneten Softwareprogrammes über die Datenverarbeitungseinrichtung 56 auch automatisch auswertbar sind.

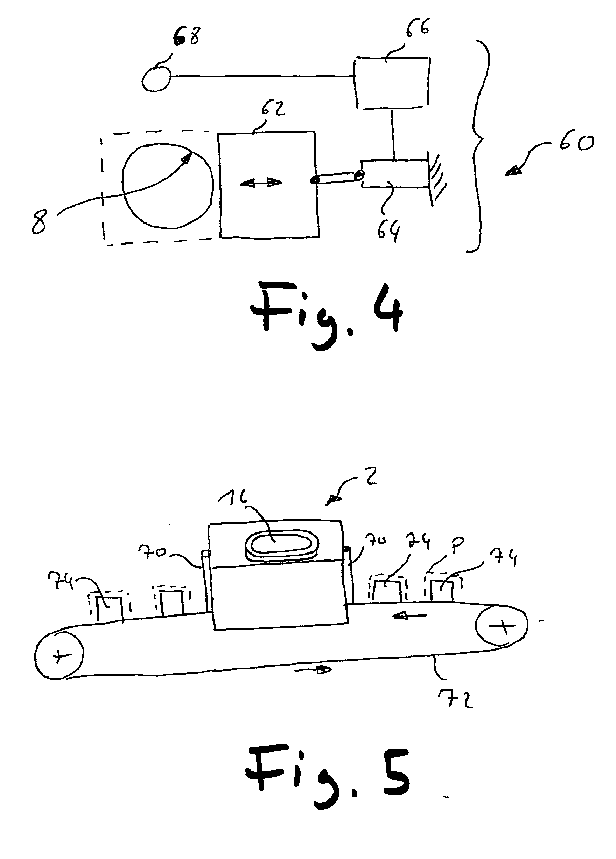

In der Fig. 4 ist eine Prinzipskizze einer für die für den Patienten bestimmten Einführungsöffnungen 8 zugeordneten Schließ- und Öffnungseinrichtung 60 zum automatischen Öffnen und Schließen der Einführungsöffnung 8 dargestellt. Diese Schließ- und Öffnungsvorrichtung 60 umfaßt Pro Einführungsöffnung 8 jeweils einen plattenförmigen Verschlußschieber 62, der durch eine Stelleinrichtung 64 in den in der Fig. 4 durch einen Doppelpfeil angedeuteten Richtungen von einer geöffneten in eine geschlossene Position (durch gestrichelte Linie angedeutet) und zurück bewegbar ist. Anstelle eines einfachen plattenförmigen Bauteils 62 sind selbstverständlich auch andere geeignete Verschlüsse, z.B. irisblendenartige Verschlüsse oder dergleichen, realisierbar. Die Stelleinrichtung 64 ist mit einer Kontrolleinrichtung 66 gekoppelt, mit der wiederum ein Sensor 68, hier ein Annäherungssensor 68, verbunden ist. Durch ein entsprechendes Signal des Sensors 68 wird der Öffnungs- und Schließvorgang des Verschlußschiebers 62 über die Kontrolleinrichtung 66 und die Stelleinrichtung 64 kontrolliert. Die Schließ- und Öffnungseinrichtung 60 gestattet insbesondere ein kontakt- bzw. berührungsloses Öffnen und Schließen der Einführungsöffnungen 8.

In der Fig. 5 ist eine dritte Ausführungsform der erfindungsgemäßen Vorrichtung dargestellt. Diese Ausführungsvariante besitzt zwei voneinander beabstandete und an jeweils gegenüberliegenden Seiten der Vorrichtung angebrachte seitliche Durchgangsöffnungen 70, durch die eine durch den aufgeleuchteten Innenraum 4 hindurchführende Förder- oder Transporteinrichtung 72 verläuft. Auf diese Weise können z.B. mit einem fluoreszierenden kosmetischen oder dermatologischen Präparate behandelte Testgegenstände 74 auf besonders rationelle Art und Weise in Serie überprüft werden. Diese Anwendung könnte zum Beispiel zur Überprufung von technischen Oberflächen genutz werden.

Es werden nun weitere Details der vorzugsweise in Verbindung mit der erfindungsgemäßen Vorrichtung verwendeten, oben bereits erwähnten Zusammensetzung zur topischen Applikation erläutert werden.

Bei dieser Zusammensetzung zur topischen Applikation ist eine Verbindung der Formel (I) bevorzugt, bei der R³ SO₃⁻M⁺, wobei M ein Alkalimetallatom, vorzugsweise Natrium oder Kalium, darstellt, insbesondere wenn diese Gruppe jeweils in 2-Stellung der Styrylgruppe vorliegt, in der erfindungsgemäßen Zusammensetzung enthalten. Am bevorzugtesten wird 4,4'-bis(2 Sulfostyryl)biphenyl-dinatriumsalz in der erfindungsgemäßen Zusammensetzung verwendet.

Die Verbindungen der Formel (I) können beispielsweise durch die in US 4 925 595, US 4 602 087, US 4 970 028 und US 4 940 555 beschriebenen Verfahren hergestellt werden.

Die Verbindungen der Formel (I) können rein oder auch als in einem Harz, beispielsweise einem Melamin-Sulfonamid-Paraformaldehyd-Harz, eingeschlossen in der erfindungsgemäßen Zusammensetzung vorliegen.

Die Distyrylverbindung der Formel (I) ist vorzugsweise in einer Menge von bis zu 2 Gew.%, besonders bevorzugt in einer Menge von 0.01 - 0.1 Gew.% in der erfindungsgemäßen Zusammensetzung enthalten.

Die erfindungsgemäßen Zusammensetzungen zur topischen Applikation schließen Cremes, Salben, Lotionen, Gele, Sprays, Öle, Hydrogele, Lösungen, Suspensionen und Pasten ein.

Erfindungsgemäß bevorzugt wird eine Zusammensetzung auf Emulsionsbasis, besonders bevorzugt eine Öl-in-Wasser (O/W)-Emulsion. Eine (O/W)-Emulsion ist insbesondere bei Verwendung wasserlöslicher Verbindungen der Formel (I) bevorzugt. Andererseits können erfindungsgemäß bei Verwendung von (W/O)-Emulsionen auch nicht-wasserlösliche Verbindungen der Formel (I) bevorzugt verwendet werden.

Die erfindungsgemäßen Zusammensetzungen zur topischen Applikation enthalten normalerweise neben dem Fluoreszenzmarker nicht toxische, pharmazeutisch annehmbare Träger einschließlich Mikrokugeln und Liposomen. Die pharmazeutisch annehmbaren Träger können Emulgatoren, Antioxidantien, Puffermittel, Konservierungsstoffe, Benetzungsmittel, Chelatbildner, gelbildende Mittel, eine Ölkomponente, Aromastoffe, Hautschutzmittel und Wasser einschließen.

Beispiele für Emulgatoren sind natürlich vorkommende Gummiarten, wie z.B. Akaziengummi und Tragantgummi, natürlich vorkommende Phosphatide wie z.B. Sojabohnenlecithin und Sorbitanmonooleat-Derivate, Unguentum Emulsificans Aquosum und höhere Alkohole wie Cetyl- oder Stearylalkohol.

Beispiele der Antioxidantien sind butylierte Hydroxyanisole, Ascorbinsäure und Derivate hiervon, Tocopherol und Derivate hiervon und Cystein.

Beispiele für Konservierungsstoffe sind Parabene und Benzalkoniumchlorid.

Beispiele für Benetzungsmittel sind Glycerin, Propylenglykol, Sorbitol und Harnstoff.

Beispiele für Chelatbildner sind Natrium-EDTA, Citronensäure und Phosphorsäure.

Beispiele für gelbildende Mittel sind Carbopol, Cellulosederivate, Betonit, Alginate und Gelatine.

Beispiele für die Ölkomponente sind Bienenwachs, Paraffin, Cetylpalmitat, Vaselin, Pflanzenöl, Sorbitanester der Fettsäuren und Ethylenoxid, z.B. Polyoxyethylen-Sorbitanmonooleat (Tween).

Beispiele für Aromastoffe sind Oleum Citri und Oleum Rosmarini.

Die Herstellung der erfindungsgemäßen Zusammensetzung kann derart erfolgen, daß der Fluoreszenzmarker der Formel (I) in eine bereits vorgefertigte Zusammensetzung, beispielsweise eine Salbe, eingearbeitet wird. Alternativ kann bei Vermischen der einzelnen Komponenten der Zusammensetzung der Fluoreszenzmarker der Formel (I) zu jedem Zeitpunkt zugefügt werden. Unabhängig von der Reihenfolge der Zugabe der einzelnen Inhaltsstoffe ist jedoch stets auf eine möglichst homogene und feine Verteilung der Fluoreszenzmarker zu achten.

Weiterhin stellt die Erfindung die Verwendung der erfindungsgemäßen Zusammensetzung zur Applikationskontrolle von Hautkosmetika, dermatologischer Externa und Desinfektionsmitteln bereit, wie z.B. Reinigungscremes, Sonnenschutzcremes, Desinfektionslösungen und insbesondere Hautschutzsalben. Weiterhin stellt die Erfindung die Verwendung der erfindungsgemäßen Zusammensetzung zur Schulung zum korrekten Waschen der Haut bereit. Ein solches Waschen schließt u.a. das Waschen mit Wasser und u.U. Seife wie auch das Waschen mit Desinfektionslösungen ein.

Besonders bevorzugt wird diese Applikationskontrolle mit einer Vorrichtung durchgeführt, wie sie in dem deutschen Gebrauchsmuster Nr. 297 19 497.6 beschreiben ist. Hierbei handelt es sich um eine Vorrichtung zum visuellen Erkennen und/oder Darstellen der Verteilung von fluoreszierenden kosmetischen, dermatologischen oder desinfizierend wirkenden Präparaten, umfassend mindestens ein hohlraumartig ausgestaltetes Gehäuse mit wenigstens einem vor äußeren Lichteinflüssen abschirmbaren Innenraum, mindestens eine im Gehäuse vorgesehene Durchgangsöffnung, die in den Innenraum mündet, mindestens eine im Innenraum des Gehäuses angeordnete Lichtquelle, die ein Licht in einem vorbestimmten Wellenlängenbereich produziert, und mindestens eine im Gehäuse angeordnete Kontrollöffnung.

Erfindungsgemäß wird vorzugsweise eine UV-Lichtquelle verwendet, die auf die Absorptionseigenschaften der Verbindung der verwendeten Formel (I) abgestimmt ist, i.a. wird jedoch eine Quecksilberhochdrucklampe verwendet.

Bei erfindungsgemäßer Verwendung wird die erfindungsgemäße Zusammensetzung zur topischen Applikation auf die Hautoberfläche, z.B. die Handoberfläche aufgetragen. Erst durch die UV-Bestrahlung mit beispielsweise der oben beschriebenen Vorrichtung tritt dann die Fluoreszenz der applizierten Zusammensetzung zu tage, wobei bei ungleichmäßiger, ungenügender bzw. inkorrekter Applikation dunkle Flecken vor dem Hintergrund der ansonsten grell weiß fluoreszierenden Hand zu erkennen sind. Wird die erfindungsgemäße Zusammensetzung zur Schulung korrekten Waschens der Haut bzw. zur korrekten Desinfektion der Haut verwendet, wird vorzugsweise zunächst die erfindungsgemäße Zusammensetzung aufgetragen, wobei die korrekte Auftragung anschließend vorteilhafterweise unter UV-Licht und durch Beobachtung der Fluoreszenz überprüft wird. Dann folgt der Waschvorgang. Ist dieser ungenügend bzw. inkorrekt, verbleibt die erfindungsgemäße Zusammensetzung auf der Haut und die ungenügend gewaschenen Hautpartien können mittels UV-Bestrahlung über die Fluoreszenz sichtbar gemacht werden.

Die erfindungsgemäße Verwendung betrifft sowohl medizinische wie auch industrielle Bereiche, so zum Beispiel bei der Anleitung und Kontrolle zur wirksamen oder korrekten Applikation von Desinfektionsmitteln oder von Hautschutzpräparaten in dermatosegefährdeten Arbeitsbereichen wie etwa in der chemischen Industrie, der Lebensmittelindustrie, in Reinigungsbetrieben, in Friseurbetrieben, in Bäckerbetrieben usw., in der medizinischen und industriellen Ausbildung und Anwendung, wie z.B. im OP-Bereich, in Reinräumen, in der Forschung, bei der medizinischen und kosmetischen Hautpflege, bei der medizinischen Überwachung kurativer dermatologischer maßnahmen, und letztendlich auch im privaten Anwendungsbereich. Die erfindungsgemäße Verwendung gestattet es insbesondere, hautgefährdete Personen zum korrekten Hautschutz zu motivieren.

### Herstellungsbeispiel:

30 Gewichtsteile Cetyl-/Stearylalkohol, 35 Gewichtsteile dickflüssiges Paraffin und 35 Gewichtsteile weißes Vaselin werden auf dem Wasserbad geschmolzen. Dann werden 0.1 Gewichtsteile 4,4-bis(2-Sulfostyryl)biphenyl-dinatriumsalz (Tinopal CBS-X, CIBA Spezialitätenchemie AG) unter Rühren hinzugefügt. Nach beendeter Zugabe werden dann die Inhaltsstoffe nochmals heftig verrührt, so daß eine möglichst homogene Öl in Wasser Emulsion erhalten wird.

Die Erfindung ist nicht auf die obigen Ausführungsbeispiele, die lediglich der allgemeinen Erläuterung des Grundgedankens der Erfindung dienen, beschränkt. Im Rahmen des Schutzumfangs können die erfindungsgemäßen Vorrichtungen vielmehr auch andere als die oben beschriebenen Ausgestaltungsformen annehmen. Die Vorrichtungen können hierbei insbesondere Merkmale aufweisen, die eine Kombination aus den jeweiligen Einzelmerkmalen der zugehörigen Ansprüche darstellt. Insbesondere können die Durchführungs- beziehungsweise Einführungsöffnungen auch andere als die oben beschriebenen Formen aufweisen. So kann eine Durchführungs- öffnungen beispielsweise auch oval, rechteckig, schlitzförmig usw. ausgestaltet sein. Sofern die Durchführungs- beziehungsweise Einführungsöffnungen zum Einführen von menschlichen Gliedmaßen vorgesehen sind, so sind die besagten Öffnungen vorzugsweise den zu untersuchenden Gliedmaßen entsprechend ergonomisch angepaßt und dementsprechend ausgestaltet. In diesem Zusammenhang sind im Sinne der Erfindung auch unterschiedlich geformte, austauschbare Öffnungseinsätze denkbar. Ein gleiches gilt für die Sichtöffnungen. Die Durchgangs- und Sichtöffnungen sowie die Lichtquellen können zudem den jeweiligen Gegebenheiten entsprechend an anderen als den oben beschriebenen Stellen positioniert sein. Des weiteren ist eine Ausrüstung der Vorrichtung mit einer internen Energiequelle, zum Beispiel einem Akkumulator, sowie die Bereitstellung von zusätzlichen Meß- und Kontrollgeräten, zum Beispiel Thermometer, Luminometer, Stoppuhren, Betriebszustandsanzeigen usw., vorgesehen. Auch die Außenform des Gehäuses beziehungsweise die Gestalt seiner einzelnen Gehäuseteile sowie die Form und Ausgestaltung des Gehäuseinnenraumes kann je nach Anwendungsfall sowie zum Erzielen bestimmter technischer und gestalterischer Effekte erheblich von den im Rahmen der Ausführungsbeispiele erörterten Varianten abweichen. Die Erfindung ist nicht ausschließlich auf UV-Lichtquellen beschränkt. Prinzipiell sind auch andere Lichtquellen und/oder Strahlungsquellen geeignet. Die oben erwähnte Spendereinrichtung für das kosmetische oder dermatologische Präparat oder andere Chemikalien kann auch außerhalb des Gehäuses angebracht sein. Obwohl die erfindungsgemäße Vorrichtung in den obigen Ausführungsbeispielen als relativ kleines mobiles beziehungsweise tragbares Gerät dargestellt wurde, ist es grundsätzlich ebenso möglich den Innenraum der Vorrichtung als begehbare mobile oder stationäre Kammer auszugestalten. Für besondere Anwendungsfälle kann die Vorrichtung überdies mit einer Klimaanlage ausgestattet sein, mit deren Hilfe die klimatischen Zustände im Gehäuseinnenraum, insbesoondere Temperatur, Luftfeuchtigkeit und so weiter, kontrollierbar sind. Obwohl die Erfindung vorstehend primär in Verbindung mit der Anwendung von fluoreszierenden kosmetischen oder dermatologischen Präparaten beschrieben wurde, ist die erfindungsgemäße Vorrichtung grundsätzlich auch in vollkommen anderen Anwendungsgebieten einsetzbar, beispielweise bei der Überprüfung der Verteilung einer mit einer fluoreszierenden Substanz versetzen Schuhcreme auf einer Lederoberfläche.

Bezugszeichen in den Ansprüchen, der Beschreibung und den Zeichnungen dienen lediglich dem besseren Verständnis der Erfindung und sollen den Schutzumfang nicht einschränken.

### Bezugszeichenliste

Es bezeichnen:
- 2: Gehäuse
- 2a: Oberes Gehäuseteil
- 2b: Mittleres Gehäuseteil
- 2c: Unteres Gehäuseteil / wannenartiger Sammel- oder Auffangbehälter
- 4: Gehäuseinnenraum
- 6: Vertikaler Frontbereich von 2
- 8: Durchgangsöffnungen / Einführungsöffnungen
- 10: UV-Lichtquelle / Strahlungsquelle
- 12: Schalteinrichtung (manuell und/oder automatisch)
- 14: Oberer abgeschrägter Frontbereich von 2
- 16: Kontrollöffnung / Sichtöffnung
- 18: Tubusartige Verkleidung von 18
- 20: Griffe
- 22: Schauglas / Lupe
- 24: UV-Lichtschutzfiltereinrichtung / Strahlungsschutz
- 26: Einwegmanschetten (auswechselbar, hygienisch)
- 28: Schwarze matte Oberfläche
- 30: Reflektierende Oberflächen
- 32: Zweite Sichtöffnung
- 34: Rechter unterer Rückseitenbereich von 2
- 36: Handschuhe
- 38: Durchgangsöffnungen (weitere)
- 40: Ablage- , Stütz-, Fixierungs-, Halterungseinrichtung
- 42: Spendereinrichtung (dosierfähig)
- 44: Innenauskleidung (auswechselbar, hygienisch)
- 46: Absaugeinrichtung
- 48: Reinigungseinrichtung
- 50: Kontrollöffnung (zusätzliche)
- 52: Bilderfassungseinrichtung / Videokamera / Meßeinrichtung
- 54: Schnittstelle
- 56: Datenverarbeitungseinrichtung
- 58: Visuellen Darstellungsvorrichtung / Monitor
- 60: Schließ- und Öffnungseinrichtung
- 62: Verschlußschieber von 60
- 64: Stelleinrichtung von 60
- 66: Kontrolleinrichtung von 60
- 68: Sensor / Annäherungssensor von 60
- 70: Seitliche Durchgangsöffnungen
- 72: Förder- oder Transporteinrichtung
- 74: Testgegenstände
- H: Hand / Gliedmaßen
- P: Fluoreszierendes bzw. phosphoreszierendes bzw. strahlungsabsorbierendes, nicht-lumineszierendes kosmetisches oder dermatologisches Präparat

## Patentansprüche

1. Vorrichtung zum visuellen Erkennen und/oder Darstellen der Verteilung von fluoreszierenden kosmetischen oder dermatologischen Präparaten (P), **umfassend**
- mindestens ein hohlraumartig ausgestaltetes Gehäuse (2) mit wenigstens einem vor äußeren Lichteinflüssen abschirmbaren Innenraum (4),
- mindestens eine im Gehäuse (2) vorgesehene Durchgangsöffnung (8, 38), die in den Innenraum (4) mündet,
- mindestens eine im Innenraum (4) des Gehäuses (2) angeordnete Lichtquelle (10), die ein Licht in einem vorbestimmten Wellenlängenbereich produziert, und
- mindestens eine im Gehäuse (2) angeordnete Kontrollöffnung (16, 32, 50).

2. Vorrichtung zum visuellen Erkennen und/oder Darstellen der Verteilung von phosphoreszierenden kosmetischen oder dermatologischen Präparaten (P), **umfassend**
- mindestens ein hohlraumartig ausgestaltetes Gehäuse (2) mit wenigstens einem vor äußeren Lichteinflüssen abschirmbaren Innenraum (4),
- mindestens eine im Gehäuse (2) vorgesehene Durchgangsöffnung (8, 38), die in den Innenraum (4) mündet,
- mindestens eine im Innenraum (4) des Gehäuses (2) angeordnete Lichtquelle (10), die ein Licht in einem vorbestimmten Wellenlängenbereich produziert, und
- mindestens eine im Gehäuse (2) angeordnete Kontrollöffnung (16, 32, 50).

3. Vorrichtung zum visuellen Erkennen und/oder Darstellen der Verteilung von strahlungsabsorbierenden, nicht-lumineszierenden kosmetischen oder dermatologischen Präparaten (P), **umfassend**
- mindestens ein hohlraumartig ausgestaltetes Gehäuse (2) mit wenigstens einem vor äußeren Lichteinflüssen abschirmbaren Innenraum (4),
- mindestens eine im Gehäuse (2) vorgesehene Durchgangsöffnung (8, 38), die in den Innenraum (4) mündet,
- mindestens eine im Innenraum (4) des Gehäuses (2) angeordnete Lichtquelle (10), die ein Licht in einem vorbestimmten Wellenlängenbereich produziert, und
- mindestens eine im Gehäuse (2) angeordnete Kontrollöffnung (16, 32, 50).

4. Vorrichtung nach Anspruch 1, 2 oder 3,
**dadurch gekennzeichnet, daß** die Lichtquelle eine UV-Lichtquelle (10) ist.

5. Vorrichtung nach einem oder mehreren der vorhergenannten Ansprüche,
**dadurch gekennzeichnet, daß**
die Durchgangsöffnung (16, 32) tubusartig ausgestaltet ist.

6. Vorrichtung nach einem oder mehreren der vorhergenannten Ansprüche,
**dadurch gekennzeichnet, daß**
die mindestens eine Durchgangsöffnung als Einsteck- oder Einführungsöffnung (8, 38) für menschliche Gliedmaßen (H) ausgebildet ist.

7. Vorrichtung nach einem oder mehreren der vorhergenannten Ansprüche,
**dadurch gekennzeichnet, daß**
die Durchgangsöffnung (8, 38) im wesentlichen lichtdicht verschließbar ausgebildet ist.

8. Vorrichtung nach einem oder mehreren der vorhergenannten Ansprüche,
**dadurch gekennzeichnet, daß**
die Durchgangsöffnung (8, 38) mit einer manuell und/oder automatisch betätigbaren Schließ- und Öffnungseinrichtung (60) zum manuellen und/oder automatischen Öffnen und Schließen der Durchgangsöffnung (8) versehen ist.

9. Vorrichtung nach Anspruch 8,
**dadurch gekennzeichnet, daß**
der Schließ- und Öffnungseinrichtung (60) eine Sensoreinrichtung (68) zum Kontrollieren des Öffnungs- oder Schließvorganges zugeordnet ist.

10. Vorrichtung nach einem oder mehreren der vorhergenannten Ansprüche,
**dadurch gekennzeichnet, daß**
wenigstens zwei voneinander beabstandete Durchgangsöffnung (8, 38) vorgesehen sind, durch die eine durch den Innenraum (4) hindurch führende Fördereinrichtung (72) verläuft.

11. Vorrichtung nach einem oder mehreren der vorhergenannten Ansprüche,
**dadurch gekennzeichnet, daß**
die Kontrollöffnung (16, 50) als eine von der Durchgangsöffnung (8, 38) beabstandete und in den Innenraum (4) mündende Sichtöffnung (16, 50) ausgestaltet ist.

12. Vorrichtung nach Anspruch 11,
**dadurch gekennzeichnet, daß**
die Sichtöffnung (16, 50) tubusartig ausgestaltet ist.

13. Vorrichtung nach Anspruch 11 oder 12,
**dadurch gekennzeichnet, daß**
die Sichtöffnung (16, 50) mit mindestens einer Lichtfilter- und/oder Lichtschutzfiltereinrichtung (24) versehen ist.

14. Vorrichtung nach einem der Ansprüche 11 bis 13,
**dadurch gekennzeichnet, daß**
die Sichtöffnung (16, 50) als optisches Instrument ausgebildet oder mit wenigstens einem solchem ausgestattet ist.

15. Vorrichtung nach einem oder mehreren der vorhergenannten Ansprüche,
**dadurch gekennzeichnet, daß**
Die Durchgangsöffnung (8, 38) und/oder die Sichtöffnung (16, 50) mit einer Öffnungsverkleidung (18, 26) versehen ist beziehungsweise sind.

16. Vorrichtung nach Anspruch 15,
**dadurch gekennzeichnet, daß**
Öffnungsverkleidung als auswechselbares hygienisches Ein- oder Mehrwegprodukt (26) ausgestaltet ist.

17. Vorrichtung nach einem oder mehreren der vorhergenannten Ansprüche,
**dadurch gekennzeichnet, daß**
der Innenraum (4) im wesentlich hermetisch lichtdicht abschließbar ist.

18. Vorrichtung nach einem oder mehreren der vorhergenannten Ansprüche,
**dadurch gekennzeichnet, daß**
der Innenraum (4) teilweise oder vollständig mit einer reflektierenden (30) und/oder diffusen Oberfläche (28) versehen ist.

19. Vorrichtung nach einem oder mehreren der vorhergenannten Ansprüche,
**dadurch gekennzeichnet, daß**
der Innenraum (4) teilweise oder vollständig mit einer auswechselbaren hygienischen Innenauskleidung (44) versehen ist.

20. Vorrichtung nach einem oder mehreren der vorhergenannten Ansprüche,
**dadurch gekennzeichnet, daß**
im Innenraum (4) wenigstens eine Spendereinrichtung (42) für ein kosmetische oder dermatologisches Präparat (P) angeordnet ist.

21. Vorrichtung nach einem oder mehreren der vorhergenannten Ansprüche,
**dadurch gekennzeichnet, daß**
der Innenraum (4) an wenigstens eine Absaugeinrichtung (46) für flüssige und/oder gasförmige Substanzen angeschlossen ist.

22. Vorrichtung nach einem oder mehreren der vorhergenannten Ansprüche,
**dadurch gekennzeichnet, daß**
im Innenraum (4) wenigstens eine Ablage- und/oder Stütz- und/oder Fixierungs- und/oder Halterungseinrichtung (40) vorgesehen und in einem Strahlungsbereich der Lichtquelle (10) positioniert und/oder durch die Sichtöffnung (16, 50) einsehbar angeordnet ist.

23. Vorrichtung nach einem oder mehreren der vorhergenannten Ansprüche,
**dadurch gekennzeichnet, daß**
das Gehäuse (2) über mindestens einen unteren Gehäuseabschnitt (2c) verfügt, der als Sammel- oder Auffangbehälter (2c) ausgebildet ist.

24. Vorrichtung nach einem oder mehreren der vorhergenannten Ansprüche,
**dadurch gekennzeichnet, daß**
diese mit wenigstens einer Reinigungseinrichtung (48) zum Reinigen des Innenraumes (4) ausgestattet ist.

25. Vorrichtung nach einem oder mehreren der vorhergenannten Ansprüche,
**dadurch gekennzeichnet, daß**
diese wenigstens eine dem Innenraum (4) zugeordnete Bilderfassungseinrichtung (52) aufweist.

26. Vorrichtung nach einem oder mehreren der vorhergenannten Ansprüche,
**dadurch gekennzeichnet, daß**
diese über wenigstens eine Meßeinrichtung (52) zum Messen der Verteilung des kosmetischen und dermatologischen Präparates (P) verfügt.

27. Vorrichtung nach einem oder mehreren der vorhergenannten Ansprüche,
**dadurch gekennzeichnet, daß**
diese über wenigstens eine Schnittstelle (54) zum Herstellen einer Verbindung zu einer internen und/oder externen Datenverarbeitungseinrichtung (56) und/oder einer visuellen Darstellungsvorrichtung (58) verfügt.

28. Vorrichtung nach einem oder mehreren der vorhergenannten Ansprüche,
**dadurch gekennzeichnet, daß**
diese modulartig aufgebaut ist, mit mindestens einem die Lichtquelle (10) und/oder die Sichtöffnung (16) aufweisenden ersten Gehäuseabschnitt (2a) sowie mindestens einem die Durchgangsöffnung (2b) aufweisenden zweiten Gehäuseteil (2b), wobei die beiden Gehäuseteile (2a, 2b) lösbar miteinander gekoppelt sind.

29. Set zur Kontrolle der Verteilung eines fluoreszierenden kosmetischen oder dermatologischen Präparates (P),
**umfassend**
- eine Vorrichtung nach Anspruch 1, und
- mindestens ein fluoreszierendes kosmetisches oder dermatologisches Präparat (P) in der Form einer Zusammensetzung zur topischen Applikation, umfassend eine Distyrylverbindung der Formel 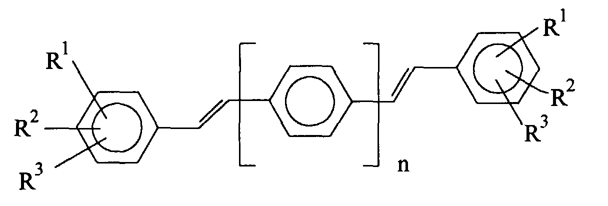 wobei R¹ und R² jeweils unabhängig voneinander ein Wasserstoffatom, ein Halogenatom oder eine C₁-C₄-Alkylgruppe darstellen; R³ ein Wasserstoffatom, ein Halogenatom, eine C₁-C₄-Alkylgruppe, eine Cyanogruppe, SO₃⁻M^{+,} wobei M⁺ ein Alkalimetallion darstellt, oder -X-Y-Z darstellt, wobei X eine Einfachbindung, ein Sauerstoffatom, ein Schwefelatom, -CONR₆ oder -COO- darstellt, wobei R₆ ein Wasserstoffatom, eine C₁-C₄-Alkylgruppe, oder Cyanoethyl darstellt, Y C₁-C₄-Alkylen oder Hydroxypropylen darstellt und Z eine quartäre Ammonium-Gruppe darstellt; und n 1 oder 2, vorzugsweise 2 ist.

30. Zusammensetzung zur topischen Applikation
**umfassend** eine Distyrylverbindung der Formel (I) 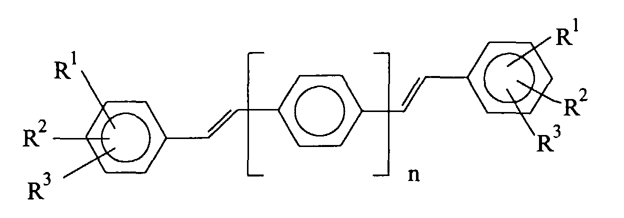 wobei R¹ und R² jeweils unabhängig voneinander ein Wasserstoffatom, ein Halogenatom oder eine C₁-C₄-Alkylgruppe darstellen; R³ ein Wasserstoffatom, ein Halogenatom, eine
C₁-C₄-Alkylgruppe, eine Cyanogruppe, SO₃⁻M^{+,} wobei M⁺ ein Alkalimetallion darstellt, oder -X-Y-Z darstellt, wobei X eine Einfachbindung, ein Sauerstoffatom, ein Schwefelatom, -CONR₆ oder -COO- darstellt, wobei R₆ ein Wasserstoffatom, eine C₁-C₄-Alkylgruppe, oder Cyanoethyl darstellt, Y C₁-C₄-Alkylen oder Hydroxypropylen darstellt und Z eine quartäre Ammonium-Gruppe darstellt; und n 1 oder 2, vorzugsweise 2 ist.

31. Verwendung der Zusammensetzung nach Anspruch 30 zur Applikationskontrolle dermatologischer Externa.

32. Verwendung der Zusammensetzung nach Anspruch 30 zur Applikationskontrolle von Hautkosmetika.

33. Verwendung der Zusammensetzung nach Anspruch 30 zur Schulung korrekten Waschens der Haut.

34. Verwendung der Zusammensetzung nach Anspruch 30 zur Schulung korrekten Desinfizierens der Haut.
